# EUROPEAN PATENT APPLICATION

(11) **EP 2 283 773 A1**
(43) Date of publication of application: **16.02.2011**
(21) Application number: 09167560.3
(22) Date of filing: 10.08.2009
(51) Int. Cl.: A61B 5/087

(54) **Processing a breathing signal**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to a system and a method for processing a breathing signal. A whistling sound detection unit (44) detects, in a breathing signal (30), a whistling sound generated by a whistle (14) that is driven by a nasal airflow of a person; and a breathing phase determination unit (40) determines a breathing phase based on the detected whistling sound. For example, the whistle (14) is positioned in the nose of the person. The breathing signal (30) is received by a microphone (12) that is positioned remote from the whistle.

## Description

### FIELD OF THE INVENTION

The invention relates to the field of processing breathing signals, in particular acoustic breathing signals, and, more specifically, to a system for processing a breathing signal and a method of processing a breathing signal.

### BACKGROUND OF THE INVENTION

The breathing signal or respiratory signal and the respiratory rate are fundamental vital signs. The respiratory rate may for example be obtained from measured respiratory waveforms. For example, respiratory waveform signals are generated through sensor electrodes externally attached to the person whose respiration is to be measured. Respiratory waveforms may also be derived from electrocardiogram (ECG) waveforms.

From US 2009/0118631 A1, a method for analyzing respiration of a subject is known. A non-contact microphone of a headset is used to generate a raw signal indicative of airflow sounds of the respiration. A frequency spectrum of the raw signal is filtered, and a temporal signal is generated by integrating the spectrum and performing a moving-average calculation. Said temporal signal is analysed, and respiratory parameters, such as a breathing period, are determined.

### SUMMARY OF THE INVENTION

Measurements, which require the placement of microphones, a headset or sensor electrodes attached to a person, are obtrusive in nature and inconvenient for the person. This is specifically important when breathing signals of a sleeping person are to be processed.

Therefore, it would be desirable to be able to process breathing signals captured by a microphone positioned remote from the breathing person.

For example, when receiving or recording breathing signals by a microphone, the microphone may be placed in the vicinity of the breathing person, e.g., 50 cm away from the breathing person. However, as the acoustic energy resulting from the breathing is generally very weak, the signal-to-noise ratio, i.e. the ratio between the respiratory signal and the noise, can be very low, making it difficult to extract a relevant respiratory parameter such as the respiratory rate from the signal.

It would be desirable to be able to provide a breathing signal that facilitates performing a noise reduction operation of the breathing signal in order to facilitate determining a respiratory parameter when capturing an acoustic breathing signal at a distance from a sleeping person.

It has been found that most microphones, which have an amplifier built inside the microphone, show a sensor noise that exhibits a low-path character due to 1/f-noise of the amplifier. Some microphones, like MEMS (micro-electromechanical system) sensors have a signal-to-noise ratio (SNR) i.e. a signal to sensor-noise ratio, of approximately 60 dB. Other, superior microphones may have an SNR range of 70 to 80 dB.

It would be desirable to provide a method or system for processing a breathing signal, which method or system facilitates providing a breathing signal with a better signal to a noise ratio.

When a respiratory rate is to be calculated from a breathing signal, such as a de-noised breathing signal, it is important that the respiratory rate is computed based on time differences between two inhales or between two exhales. However, as breathing sound patterns captured by a microphone can show inhales, exhales, or both, it would be desirable to be able to determine the breathing phase or respiratory phase, i.e. detect whether at a specific time, a breathing sound is produced by an inhale or an exhale.

However, inspiratory and expiratory sounds can be very similar regarding their time-frequency characteristics. Therefore, it would be desirable to provide a system or method for processing a breathing signal that facilitates discriminating inhales and exhales.

To be better address one or more of these concerns, in a first aspect of the invention, a system for processing a breathing signal is provided, comprising:
- a whistling sound detection unit for detecting in a breathing signal a whistling sound generated by a whistle that is driven by a nasal airflow of a person; and
- a breathing phase determination unit for determining a breathing phase based on the detected whistling sound.

For example, the whistling sound has a whistle specific frequency pattern. For example, the frequency pattern is dependent on the direction of airflow through the whistle.

Preferably, the whistling sound is clearly distinguishable from naturally occuring breathing sounds. It has been found that the spectral characteristics of natural breathing sounds show relevant information up to 2 kHz. For example, said whistling sound contains at least one frequency that is above 2 kHz, preferably above 4 kHz, more preferably above 8 kHz, e.g. an ultrasonic frequency. For example, said frequency is a whistle specific frequency. For example, said frequency is dependent on the direction of airflow through the whistle. For example, said whistling sound contains an approximately constant base frequency, i.e. a base frequency that varies only in a small frequency range. Such variations may, for example, be due to different intensities of nasal airflow generating the whistling sound. For example, said base frequency is above 2 kHz, preferably above 4 kHz, more preferably above 8 kHz, e.g. an ultrasonic frequency. For example, said base frequency is a whistle specific base frequency. That is, the whistle may be tuned to the base frequency. For example, said frequency or base frequency may be above a frequency range of naturally occurring wheezing. For example, said base frequency is dependent on the direction of airflow through the whistle.

The term "breathing phase" is to be understood as comprising an inhale phase and an exhale phase. For example, a breathing sequence of a breathing person may comprise alternately inhale phases and exhale phases, which may for example be separated by neutral phases or phases of indifferent breathing or airflow.

For example, the system may comprise a processing unit, which comprises the whistling sound detection unit and the breathing phase determination unit.

For example, the system further comprises at least one whistle for positioning in a nasal airflow of a person, said whistle comprising at least one nasal airflow drivable whistling sound generator.

For example, the system further comprises at least one microphone for receiving a breathing signal that comprises a whistling sound of the whistle.

For example, the whistle is adapted for being positioned in front of and/or in the nose of the person.

For example, in order to determine a breathing phase of a person, the whistle is placed in the nose of a sleeping person. Preferably, the whistle does not block the breathing of the person through the nose, as it is known that breathing through the nose is important during sleep. Preferably, the whistle sticks out of the nose only to a limited extent. If a whistle would stick out of the nose too much, this could wake up the sleeper when moving the head.

The whistling sound forms a part of the breathing signal and has the same or similar time characteristics as the nasal airflow of the person. Thus, from the whistling sound, characteristics of the nasal airflow and, thus, of the breathing of the person may be determined.

For example, the generation of the whistling sound is dependent on the direction of airflow through the whistle, i.e. direction of the nasal airflow. For example, the whistling sound may be generated only by an inhale airflow or may be generated only by an exhale airflow. Alternatively, for example, different whistling sounds may be generated for an inhale airflow and an exhale airflow. That is, different whistling sounds may be generated dependent on the direction of airflow through the whistle. Preferably, the whistle is adapted to generate a whistling sound driven by an inhale airflow and/or an exhale airflow of the person.

When the whistle is positioned in the nasal airflow of the person, with each inhale and/or exhale, a specific whistling sound may be generated. This whistling sound is received by the microphone and processed by the whistling sound detection unit.

Providing for detection of the whistling sound of a whistle driven by the nasal airflow has the advantage that the signal-to-noise ratio of the relevant part of the breathing signal can be increased drastically. Without using a whistle, a breathing sound is, depending on the acoustic situation, often weaker or much weaker than the noise, such as sensor noise of the microphone or acoustic noise. This renders the determination of a respiratory rate very difficult or even impossible. However, by providing the whistle, the signal-to-noise ratio of the breathing signal is improved, thus facilitating determining a breathing phase or a breathing parameter, such as the respiratory rate.

Another advantage is that a microphone for receiving the breathing signal may be placed at some distance from the sleeping person. Thus, the sleeping person does not have to wear a microphone. Thus, it is not necessary that the sleeping person would wear a microphone transmitting the signal via wires, which would be disturbing for the sleeper. It is neither necessary to provide for a wireless transmission of a microphone signal. Thus, it can be avoided that a detection of the breaths or the breathing phase would be interrupted, for example, by batteries running empty.

Another advantage of providing for detection of a whistling sound of a nasal airflow driven whistle is that the time-frequency characteristics of the breathing signal are, at least partly, determined by the whistling sound(s) of the whistle. Therefore, the time-frequency characteristics are substantially stationary over an entire night, for example. The a-priori knowledge of the stationarity may be exploited by a breathing signal processing unit or a processing algorithm, thereby facilitating an improved de-noising or noise reduction and an improved detection of breathing and breathing phase.

For example, the whistle may be constructed similar to a conventional whistle producing acoustic sounds, preferably high frequency acoustic sounds or, in particular, ultrasonic acoustic sounds.

Thus, in one embodiment, the whistling sound detection unit is adapted for detecting in a breathing signal said whistling sound, said breathing signal being received by a microphone while the microphone is positioned remote from the whistle and/or the person.

For example, the whistling sound generator is adapted to be solely powered by nasal airflow. Thus, there is no power source required for the whistle such as batteries or the like. Thus, the whistling sound generating is driven and powered by the nasal airflow.

In one embodiment, the system further comprises a noise reduction unit for performing a noise reduction operation on the breathing signal. For example, an output of the microphone is coupled to an input of the noise reduction unit. For example, an output of the noise reduction unit is coupled to an input of the whistling sound detection unit.

In one embodiment, the system further comprises a breath detection unit for detecting a breath based on the breathing signal. For example, an output of the noise reduction unit may be coupled to an input of the breath detection unit. For example, an output of the breath detection unit may be coupled to an input of the breathing phase determination unit.

For example, a processing unit of the system may comprise the noise reduction unit and/or the breath detection unit.

In one embodiment, the whistling sound is an ultrasonic whistling sound. For example, the whistling sound generator is adapted to generate an ultrasonic whistling sound. In particular, the whistling sound generator may be an ultrasonic whistling sound generator.

For example, the breathing phase determination unit may be adapted for generating a breathing phase signal, i.e. a signal indicative of a currently determined breathing phase. For example, the breathing phase signal may take at least a value representing an inhale phase and a further value representing an exhale phase.

In one embodiment, the system further comprises a breathing parameter determination unit for determining a breathing parameter based on a breathing phase signal generated by the breathing phase determination unit. Said breathing phase signal may be an internal signal and may be represented, for example, in a memory of a processing unit, such as a processor register. The breathing parameter may, for example, be a breathing period and/or a breathing rate or respiratory rate. For example, the system may comprise a breathing rate determination unit for determining a breathing rate based on detecting a repeat pattern of the whistling sound in the breathing signal.

In one embodiment, the breathing phase determination unit is adapted for discriminating inhale and exhale breathing phases based on different frequency patterns of corresponding whistling sounds. For example, the whistle is adapted for generating inhale and exhale whistling sounds of different specific frequencies, the frequencies being dependent on the direction of airflow through the whistle. This facilitates discriminating breathing phases of inhales and exhales from the time-frequency characteristics of the whistling sound contained in the breathing signal.

For example, the whistling sound detection unit is adapted for using spectral analysis of the breathing signal for detecting the whistling sound. Spectral analysis allows to reliably and easily detect a whistle specific frequency pattern of the whistling sound in the breathing signal. For example, the whistling sound detection unit may be adapted for detecting the whistling sound based on spectral analysis of the breathing signal e.g. as received by the microphone.

For example, the system further comprises a spectral filter for filtering the breathing signal. For example, an output of the filter is coupled to an input of the noise reduction unit or the whistling sound detection unit.

In one embodiment, the breathing phase determination unit is adapted for discriminating breathing phases of at least two persons based on different frequency patterns of whistling sounds of whistles used by the persons. For example, the system comprises at least two whistles for positioning in respective nasal airflows of at least two persons, and the whistles are adapted for generating whistling sounds of different, whistle specific frequency patterns. Thus, the breathing phase and/or breathing parameters of multiple persons may be determined at the same time. For example, in the case of two persons, two whistles are used which are tuned to produce frequencies in different frequency ranges, e.g. having different base frequencies. The system may, for example, be applied for processing breathing signals of two bed-partners or of multiple persons in a hospital scenario.

In a further aspect of the invention, there is provided a method of processing a breathing signal, comprising the steps of:
- performing a whistling sound detection operation on a breathing signal for detecting in the breathing signal a whistling sound generated by a whistle that is driven by a nasal airflow of a person; and
- performing a breathing phase determination operation based on the detected whistling sound.

For example, the method further comprises:
- generating a whistling sound by a whistle that is positioned in a nasal airflow of a person, said generating being driven by the nasal airflow; and
- receiving a breathing signal that comprises the whistling sound of the whistle by a microphone.

Thus, a breathing phase may be determined based on the whistling sound received by the microphone.

In one embodiment, the method further comprises the step of determining a breathing parameter based on determining the breathing phase. For example, the method may comprise performing a breathing rate determination operation based on detecting a repeat pattern of the whistling sound in the breathing signal.

In one embodiment, the whistling sound detection operation is performed for detecting whistling sounds generated by at least two different whistles having different frequency patterns and driven by a nasal airflow of at least two different persons, and the breathing phase determination operation is performed for each of the at least two different whistling sounds.

For example, the method further comprises the steps of:
- performing a whistling sound detection operation on the breathing signal for detecting in the breathing signal a further whistling sound generated by a further whistle that is driven by a nasal airflow of a further person; and
- performing a further breathing phase determination operation based on the detected further whistling sound

For example, the method may comprise:
- generating a further whistling sound by a further whistle that is positioned in a nasal airflow of a different person, said generating being driven by the nasal airflow;
- receiving a breathing signal that contains the further whistling sound by the microphone.

Alternatively, said further whistling sound may be received by a further microphone, and a breathing phase of the further person is determined based on the whistling sound received by said further microphone. Thus, independent systems may be used for processing breathing signals of multiple persons, wherein whistles of the respective systems are adapted for generating whistling sounds of different, whistle specific and, thus, system specific frequencies.

For example, the method further comprises the step of performing a noise reduction operation on the breathing signal.

For example, the method further comprises the step of detecting a breath based on the breathing signal.

For example, the microphone may also receive a breathing sound generated by the breathing person and different from the whistling sound generated by the whistle. For example, a breathing signal comprising said breathing sounds as well as the whistling sound generated by the whistle may be processed by a processing unit, such as the breath detection unit. Thus, the whistling sound contained in the breathing signal may facilitate processing or analyzing the breathing signal.

These and other aspects of the invention will be apparent from and illustrated with reference to the embodiments described hereinafter.

### BRIEF DESCRIPTION OF THE DRAWINGS

- Fig. 1: schematically illustrates a setup of a system for processing a breathing signal of a person.
- Fig. 2: schematically illustrates a whistle positioned in the nose of the person.
- Fig. 3: schematically illustrates a whistle for generating acoustic signals of different specific frequency patterns dependent on the direction of airflow.
- Fig. 4: schematically illustrates a configuration of the system for processing breathing signals for use with the whistle.

### DETAILED DESCRIPTION OF EMBODIMENTS

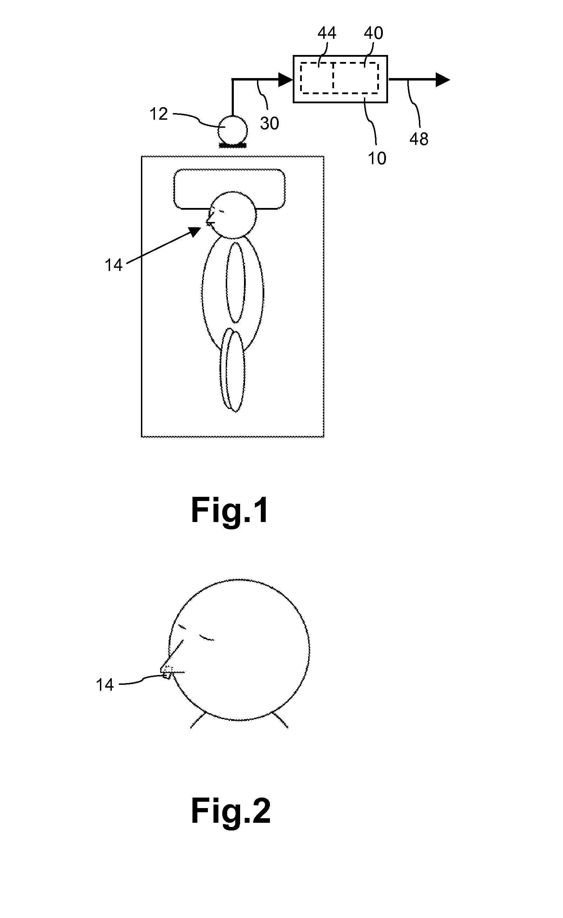

Fig. 1 shows a system for processing breathing signals comprising a processing unit 10, a microphone 12 connected to the processing unit 10 and a whistle 14 being positioned in the nose of a sleeping person. For example, the processing unit may be a part of a computer or a computer program for being executed on a computer.
Fig. 2 shows the whistle 14 being positioned in the nose of the sleeping person. The whistle 14 sticks out of the nose only to a limited extent in order not to disturb the sleeping person when moving his or her head. In particular, there is no wire or wireless connection of the whistle 14 to the microphone 12 or processing unit 10.

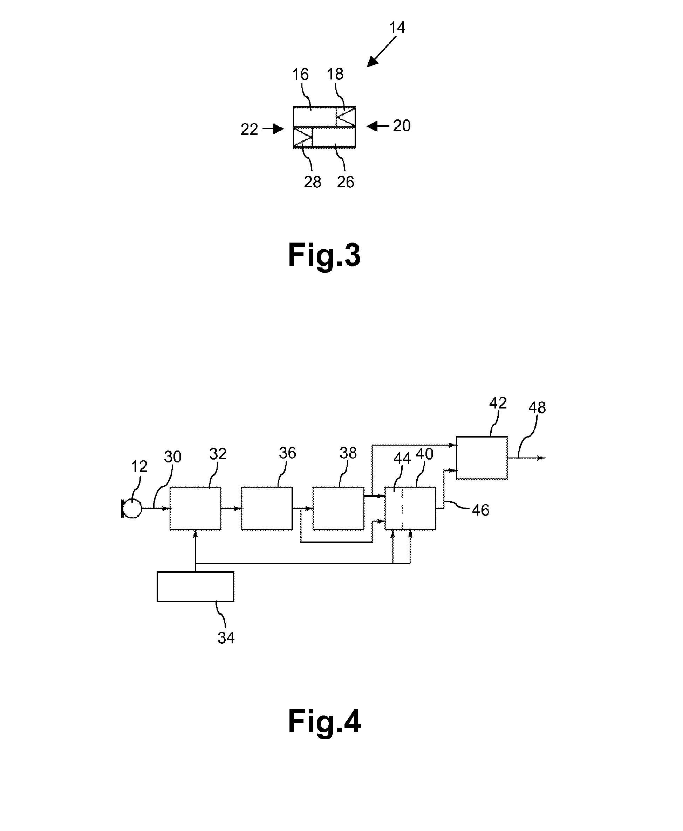

Fig. 3 exemplarily shows a possible configuration of the whistle 14. For example, the whistle 14 comprises a first whistling sound generator 16 in line with or integrally formed with or forming a valve 18 for generating a first whistling sound when air flows through the whistle 14 from a first end 20 to a second end 22. For example, the whistle 14 is inserted with the first end 20 into the nose of the person.
For example, the whistle 14 comprises a second whistling sound generator 26 and a second valve 28 configured in a similar manner as the first whistling sound generator 16 and valve 18. However, the valve 28 and, optionally, the second whistling sound generator 26 are oriented in an opposite direction to the first valve 18. That is, the second whistling sound generator 26 generates a second whistling sound, when air flows through the whistle 14 in the opposite direction.

The first and second whistling sounds have different specific frequency patterns, in particular, for example, different base frequencies.

When the whistle 24 is inserted with the first end 20 in the nose of the person, the first whistle generator 16 generates first whistling sounds corresponding to exhale breathing phases, and the second whistling sound generator 26 generates second whistling sounds corresponding to inhale breathing phases of the person.

For example, the first and second whistling sounds are high frequency acoustic sounds, in particular, ultrasonic sounds.

Fig. 4 exemplarily shows the configuration of the processing unit 10 connected to the microphone 12. In Fig. 4, a signal or data flow is schematically illustrated by arrows.

A breathing signal 30 captured by the microphone 12, which is positioned at a distance of e.g. 50 cm from the sleeping person, is input to a spectral filter 32. For example, the signal 30 may be sampled and provided block-wise to the spectral filter 32.

The spectral filter 32 performs a spectral filtering of the breathing signal 30 based on whistle parameters 34, such as information about a typical frequency pattern or frequency spectrum of the first and second whistling sounds, for example, information about the base frequencies of the whistle 14.

An output of the spectral filter 32 is coupled to an input of a noise reduction unit 36, which performs a noise reduction operation. For example, the noise reduction operation uses spectral subtraction.

An output of the noise reduction unit 36 is coupled to inputs of a breath detection unit 38 and a breathing phase determination unit 40.

The breath detection unit 38 performs a breath detection operation in order to detect moments at which a breathing sound or a whistling sound associated with a breath is present in the breathing signal 30. The breath detection unit 38 generates a breath detection signal, which is input to the breathing phase determination unit 40 and a breathing rate determination unit 42.

The breathing phase determination unit 40 comprises a whistling sound detection unit 44. From the de-noised signal output by the noise reduction unit 36, and from the breath detection signal output by the breath detection unit 38, and based on the whistle parameters 34, the whistling sound detection unit 44 detects first and second whistling sounds associated with an inhale or exhale breath. Based on the different frequency patterns, the breathing phase determination unit 40 generates a breathing phase signal 46 which is output to the breathing rate determination unit 42.

The breathing rate determination unit 42 determines the breathing rate by computing the time difference of two consecutive inhale breathing phases or two consecutive exhale breathing phases. A breathing rate signal 48 is output by the breathing rate determination unit 42.

In a further embodiment, two whistles 14, the whistling sounds of which have different specific frequency patterns, may be used for determining breathing rates of two persons simultaneously. Similar to the setup in Fig. 1, each person has a respective whistle 14 inserted into his or her nose. For example, the breathing signals are received by a common microphone 12. The processing unit 10 for determining the breathing rates of the persons may be configured similar to the system of Fig. 4. For example, two breath detection signals may be output from the breath detection unit 38, and the breathing phase detection unit may provide a breathing phase signal 46 for each person to the breathing rate determination unit 42, using the whistle parameters of the associated whistle. Alternatively, for example, the units 36, 38, 40 and 42 may be provided separately for each person and whistle, and the spectral filter 32 may have two outputs coupled to respective noise reduction units 36.

While the invention has been illustrated and described in detail in the drawings and foregoing description, such illustration and description are to be considered illustrative or exemplary and not restrictive. The invention is not limited to the disclosed embodiments.

In particular, each described feature of the method according to the invention may be advantageously used with the system according to the invention and vice versa.

Variations to the disclosed embodiments can be understood and effected by those skilled in the art in practicing the claimed invention, from a study of the drawings, the disclosure and the appended claims.

In the claims, the word "comprising" does not exclude other elements or steps, and the indefinite article "a" or "an" does not exclude a plurality. Any reference signs in the claims should not be construed as limiting the scope.

## Claims

1. A system for processing a breathing signal, comprising:
- a whistling sound detection unit (44) for detecting in a breathing signal (30) a whistling sound generated by a whistle (14) that is driven by a nasal airflow of a person; and
- a breathing phase determination unit (40) for determining a breathing phase based on the detected whistling sound.

2. The system as claimed in claim 1, further comprising;
- at least one whistle (14) for positioning in a nasal airflow of a person, said whistle (14) comprising at least one nasal airflow drivable whistling sound generator (16; 26);
- at least one microphone (12) for receiving a breathing signal (30) that comprises a whistling sound of the whistle (14).

3. The system as claimed in claim 2, wherein the whistling sound generator (16; 26) is adapted to be solely powered by nasal airflow.

4. The system as claimed in any one of the preceding claims, further comprising a noise reduction unit (36) for performing a noise reduction operation on the breathing signal (30).

5. The system as claimed in any one of the preceding claims, further comprising a breath detection unit (38) for detecting a breath based on the breathing signal (30).

6. The system as claimed in any one of the preceding claims, wherein the whistling sound is an ultrasonic whistling sound.

7. The system as claimed in any one of the preceding claims, further comprising a breathing parameter determination unit (42) for determining a breathing parameter based on a breathing phase signal (46) generated by the breathing phase determination unit (40).

8. The system as claimed in any one of the preceding claims, wherein the breathing phase determination unit (40) is adapted for discriminating inhale and exhale breathing phases based on different frequency patterns of corresponding whistling sounds.

9. The system as claimed in any one of the preceding claims, wherein the whistling sound detection unit (44) is adapted for using spectral analysis of the breathing signal (30) for detecting the whistling sound.

10. A method of processing a breathing signal, comprising the steps of:
- performing a whistling sound detection operation on a breathing signal (30) for detecting in the breathing signal (30) a whistling sound generated by a whistle (14) that is driven by a nasal airflow of a person; and
- performing a breathing phase determination operation based on the detected whistling sound.

11. The method as claimed in claim 10, further comprising the steps of:
- generating a whistling sound by a whistle (14) that is positioned in a nasal airflow of a person, said generating being driven by the nasal airflow; and
- receiving a breathing signal (30) that comprises the whistling sound of the whistle (14) by a microphone.

12. The method as claimed in claim 10 or 11, further comprising the step of:
- determining a breathing parameter based on determining the breathing phase.

13. Computer program or Computer program product for performing the method as claimed in any one of claims 10 to 12 when executed on a computer.

14. Data Carrier including a Computer program for performing the steps of the method as claimed in any one of claims 10 to 12.

15. Computer for executing a computer program as claimed in claim 13.
